(19) Europäisches Patentamt
European Patent Office
Office européen des brevets

(11) **EP 1 930 037 B1**

(12) **EUROPEAN PATENT SPECIFICATION**

(45) Date of publication and mention
of the grant of the patent:
**15.09.2010 Bulletin 2010/37**

(51) Int Cl.:
*A61M 5/145* *(2006.01)*    *A61M 5/315* *(2006.01)*

(21) Application number: **07254684.9**

(22) Date of filing: **03.12.2007**

(54) **Syringe assembly and an infusion pump assembly incorporating such**

Spritzenanordnung und diese enthaltende Infusionspumpenanordnung

Ensemble de seringue et ensemble de pompe à perfusion le comprenant

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **04.12.2006 US 633160**

(43) Date of publication of application:
**11.06.2008 Bulletin 2008/24**

(73) Proprietor: **Animas Corporation**
**West Chester, PA 19380 (US)**

(72) Inventor: **Sharifi, Bahram**
**Schwenksville, PA 19743 (US)**

(74) Representative: **Tunstall, Christopher Stephen et al**
**Carpmaels & Ransford**
**One Southampton Row**
**London**
**WC1B 5HA (GB)**

(56) References cited:
WO-A-02/04049        WO-A-99/55401
DE-U1- 20 011 366    US-A- 2 895 773

## Description

FIELD OF THE INVENTION

The present invention relates to an infusion pump assembly for controlled delivery of a pharmaceutical product to a subject, and more specifically to a syringe assembly for use with the infusion pump.

BACKGROUND OF THE INVENTION

Infusion pumps provide a significant lifestyle benefit for Individuals requiring multiple deliveries of volumetrically proportioned medication to their body over a period of time. Infusion pumps reliably dispense the required medication to the patient through an infusion path established between the patient and the pump. The infusion path is a conduit secured to the pump at one end and secured intravenously or subcutaneously to a patient on the other. The operation of the infusion pump is controlled by a processor. The processor controls the delivery of periodic dosages of medication to a patient at predetermined times. Thus, a patient is able to rely on the infusion pump for delivering the required dosage of medication intravenously or subcutaneously over a period of time. In this way, the patient need not interrupt life activities for repeated manual delivery of required medication.

As is known, infusion pumps often employ a piston-type drive mechanism for urging the contents of a pharmaceutical cartridge or "syringe assembly" internal to the pump along the infusion path to the subject. The piston-type drive selectively drives the syringe plunger to dispense a desired amount of fluid from the syringe housing.

Documents US 2895773, DE 20011366 U1 and WO 99/55401 disclose syringe assemblies with various plunger/piston-rod connections which are detachable and/or attachable outside the syringe barrel, i.e. before introducing the plunger/piston-rod combination into the syringe barrel.

WO 02/04049 discloses an injector for use with a syringe assembly with a releasable plunger/piston-rod connection that can be released/engaged at any position of the plunger/piston-rod inside the syringe barrel.

SUMMARY OF THE INVENTION

The invention is defined in claim 1.

In accordance with an aspect of the invention, a syringe assembly for use with an Infusion pump having a drive piston is provided. The syringe assembly comprises a substantially hollow syringe housing and a plunger axially movable within the syringe housing to expel a fluid therefrom. radially elastic sealing member is configured to engage a portion of the drive piston to seal the plunger relative to the drive piston.

In accordance with another aspect of the invention, an infusion pump assembly is provided. The infusion pump assembly comprises an insulin pump including a drive piston and a syringe assembly as described above including a substantially hollow syringe housing and a plunger axially movable within the syringe housing to expel a fluid therefrom. The plunger has a body with a portion configured to engage the drive piston. A sealing elastic member is positioned between the plunger body and the drive piston to seal the plunger body relative to the drive piston.

In accordance with another aspect of the invention, the plunger is configured to receive the drive piston with preferably annular gap between the drive piston and the plunger hollow body and the sealing elastic member is positioned within the gap to seal the plunger relative to the drive piston.

BRIEF DESCRIPTION OF THE DRAWINGS

The foregoing summary, as well as the following detailed description of preferred embodiments of the invention, will be better understood when read in conjunction with the appended drawings. For the purpose of illustrating the invention, there is shown various embodiments.

In the drawings:

Fig. 1 is side elevation view of an exemplary infusion pump incorporating an embodiment of the present invention, the infusion pump having a wall of its casing removed to show the layout of the components therein;

Fig. 2 is a side elevation view of the infusion pump shown in Fig. 1 with the wall in place and the pump door open;

Fig. 3 is an end elevation view of the infusion pump with the pump door open for loading a syringe assembly;

Fig. 4 is an isometric view of a syringe assembly in accordance with an exemplary embodiment of the present invention;

Fig. 5 is a an exploded cross-sectional view of the syringe assembly of Fig. 4 illustrating the sealing insert prior to assembly;

Fig. 6 is a cross-sectional view along the line 6-6 in Fig. 4;

Fig. 7 is a cross-sectional view similar to Fig. 6 of a syringe assembly in accordance with an alternative exemplary embodiment of the present invention;

Fig. 8 is a cross-sectional view of a drive piston in accordance with an exemplary embodiment of the invention;

Fig. 9 is a side elevation view in partial section illustrating insertion of the syringe assembly into the infusion pump casing;

Fig. 10 is a cross-sectional view illustrating the syringe assembly of Fig. 9 loaded in an operational position within the infusion pump;

Fig. 11 is a cross-sectional view of a drive piston in accordance with an alternative exemplary embodiment of the invention;

Fig. 12 is a cross-sectional view of a drive piston in accordance with another alternative exemplary embodiment of the invention; and

Fig. 13 is a cross-sectional view similar to Fig. 6 of a syringe assembly in accordance with another alternative exemplary embodiment of the present invention.

DETAILED DESCRIPTION OF THE INVENTION

Certain terminology is used in the following description for convenience only and is not limiting. The words "right," "left," "lower" and "upper" designate directions in the drawings to which reference is made. The words "inwardly" and "outwardly" refer to directions toward and away from, respectively, the longitudinal axis of the syringe barrel. The terminology includes the words specifically mentioned above, derivatives thereof and words of similar import.

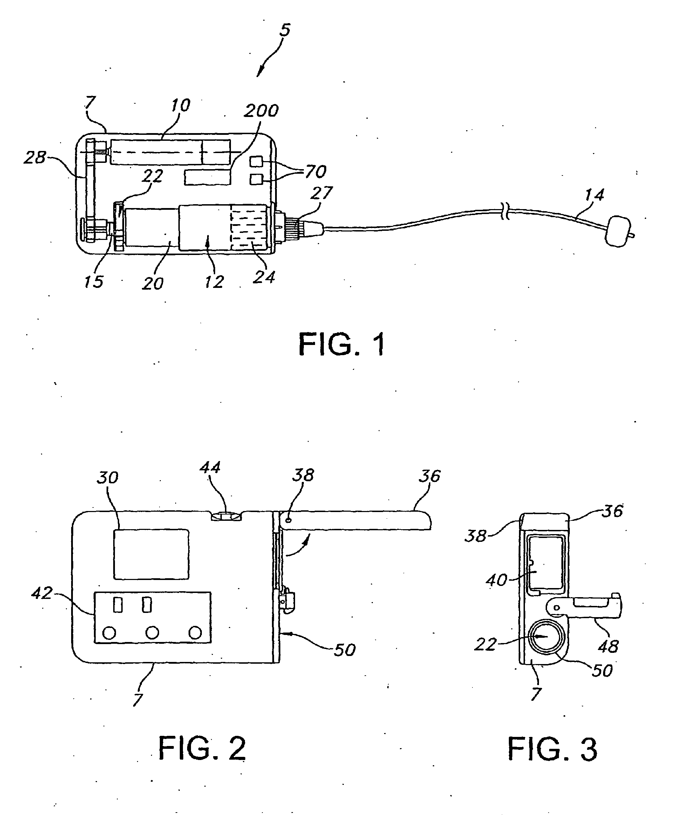

Referring to Fig. 1, piston-type infusion pump 5 in accordance with an exemplary embodiment of the present invention is shown for delivering medication 24 or other fluid (in phantom) to a patient along infusion path 14. Infusion pump 5 desirably includes sealed pump casing 7, processing circuitry 200, power cells 70, motor 10, gear train 28, lead screw 15, and drive piston 22. Syringe assembly 12 is positional within pump casing 7 such that drive piston 22 engages plunger 20 of syringe assembly 12.

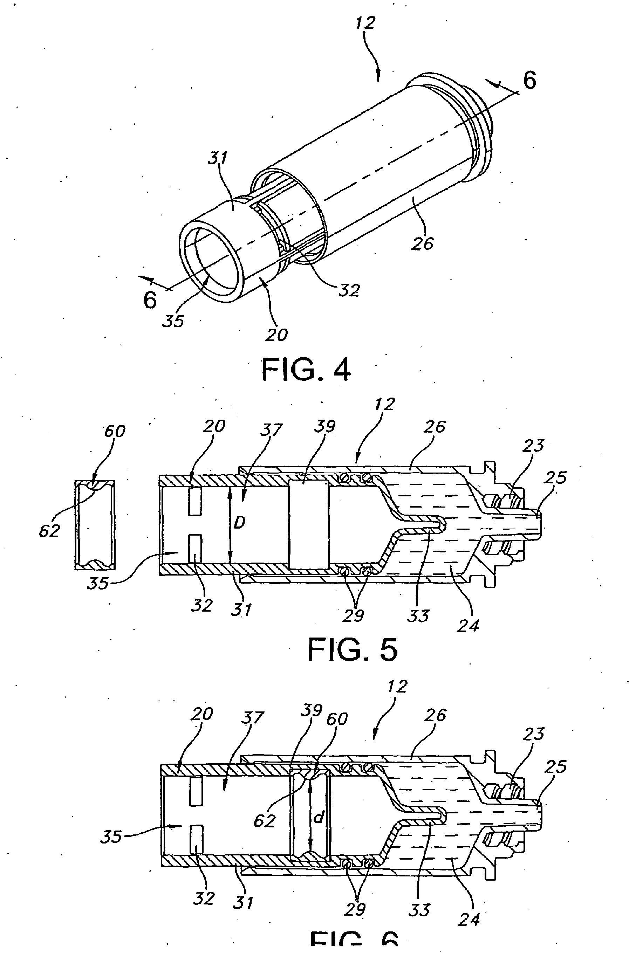

In operation, processing circuitry 200, powered by power cells 70, controls the operation of infusion pump 5. Motor 10 is incrementally engaged to infuse medication to a patient at predetermined intervals. Upon engagement, motor 10 causes lead screw 15 to rotate by means of gear train 28. When lead screw 15 is driven by motor 10, drive piston 22 is driven axially toward syringe assembly 12, thereby pushing plunger 20. This causes delivery of medication 24 from within syringe housing 26 of syringe assembly 12. Infusion path 14 is connected by connector 27 to dispensing tip 25 (see Fig. 5) of syringe housing 26 to provide fluidic communication between infusion pump 5 and a patient.

Referring now to Figs. 2-3, casing 7 of infusion pump 5 is shown. Pump casing 7 is desirably formed of a thermoplastic material and preferably made watertight by sealing any openings therein. Desirably, pump casing 7 supports LCD display 30, keypad 42, priming button 44, battery door 40, hinge 38, pump door latch 48, and pump door 36. To load a syringe assembly 12 within casing 7 pump door 36 is opened to expose the interior infusion port 50 and battery door 40. Syringe assembly 12 is configured to be moved axially through interior infusion port 50 into the interior of pump casing 7 such that plunger 20 axially engages drive piston 22, as will be described in more detail hereinafter. In Figs. 2 and 3, pump door latch 48 has been rotated away from pump casing 7 in order to release pump door 36 so it may pivot open at hinge 38. Battery door 40 is removable for replacing the power cells 70.

The present invention is not limited to the illustrated infusion pump. Infusion pump 5 may have various configurations including various controls, power sources, drive means, access ports and doors, sizes and shapes.

Referring to Figs. 4-6, syringe assembly 12 includes plunger 20 and syringe housing 26. Seal rings 29 or the like may be provided between plunger 20 and syringe housing 26 to seal medication 24 within syringe housing 26. Upon inward axial motion of plunger 20 relative to syringe housing 26, medication 24 is dispensed through dispensing tip 25. Connection interface 23 is provided adjacent dispensing tip 25 to facilitate connection to connector 27. Various connection interfaces 23 may be provided.

In the present embodiment, plunger 20 includes tubular body 31 which has a generally hollow interior 37 extending between closed end 33 and generally open end 35. Generally open end 35 is configured to receive drive piston 22 as will be described hereinafter. Plunger body 31 may include vent holes 32 or the like to allow pressure to escape as drive piston 22 is advanced into hollow interior 37. Interior annular groove 39 is provided along the interior surface of plunger body 31 and is configured to receive sealing insert 60 including elastic sealing member 62. With sealing insert 60 positioned in annular groove 39, elastic sealing member 62 extends radially inward into hollow interior 37 and has an internal diameter d which is less than the interior diameter D of tubular body 31. Interior annular groove 39 is desirably provided proximate generally open end 35, but may otherwise be positioned. Syringe assembly 12 as illustrated in Figs. 4 and 6 is pre-filled with medication 24 or the like and is ready for use in infusion pump 5.

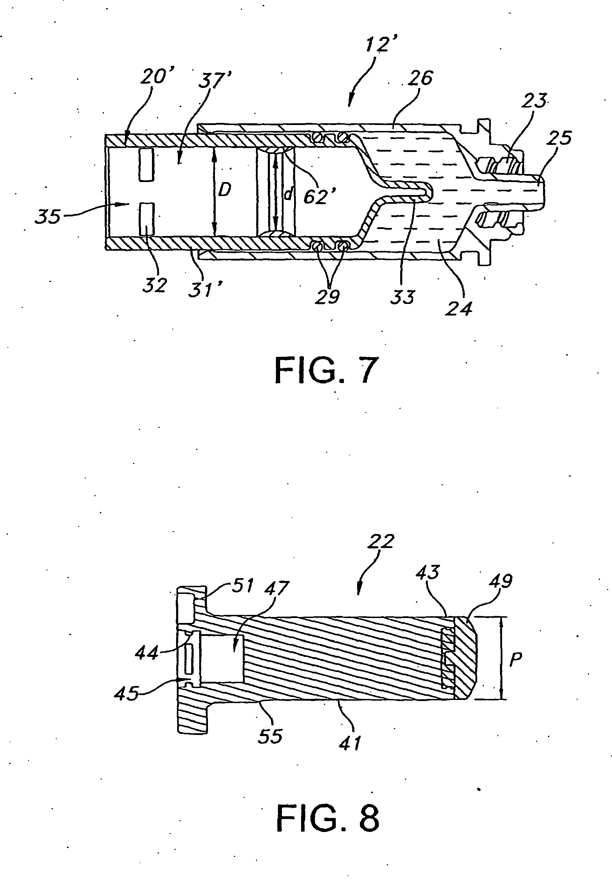

Referring to Fig. 7, syringe assembly 12' in accordance with an alternative exemplary embodiment of the present invention is shown. Syringe assembly 12' is substantially the same as in the previous embodiment, however, elastic sealing member 62' is formed integrally, for example, via overmolding or the like, within hollow interior 37' of plunger tubular body 31'. As such, an annular groove or the like is not required. In other respects, plunger 20' is substantially the same as in the previous embodiment. Elastic sealing member 62, 62' may be provided within plunger 20, 20' in various other manners other than those described herein

Referring to Figs. 8-10, drive piston 22 includes tubular body 41 having a hollow interior portion 47 which is configured to receive lead screw 15. Threads 44 adjacent open end 45 of piston body 41 are configured to engage threads 16 of lead screw 15 to facilitate driving motion of drive piston 22. Drive piston 22 is supported within casing 7, either by lead screw 15 alone or via other support structures (not shown). Shoulder 51 may be provided about open end 45 to further support drive piston 22 within casing 7. End 43 of piston body 41 is configured to receive cap 49 which is desirably manufactured from an elastomeric material. Alternatively, end 43 may be manufactured as a closed end or cap 49 may be formed integrally therewith, for example, via overmolding. Between ends 43 and 45, piston body 41 may expand radially outwardly to define shoulder 55 which is configured to engage open end 35 of plunger 20 upon insertion therein. Forward of shoulder 55, piston body 41 has an outer diameter P which is larger than interior diameter d of elastic sealing member 62.

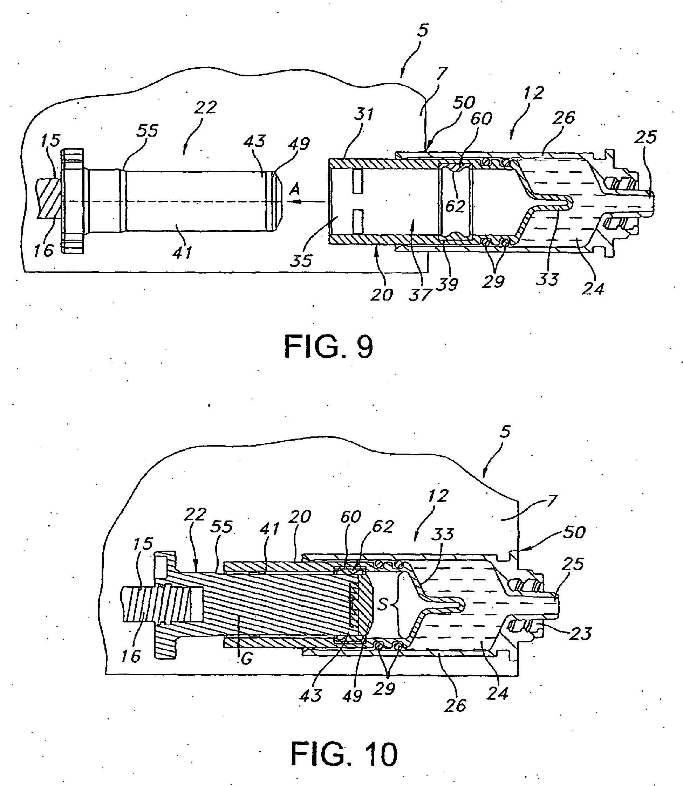

As illustrated in Fig. 9, to install syringe assembly 12 within infusion pump 5, syringe assembly 12 is moved axially through interior infusion port 50, as indicated by arrow A. End 43 of drive piston 22 enters plunger 20 through open end 35 and passes through hollow interior 37 until shoulder 55 engages open end 35. Alternatively, plunger 20 and drive piston 22 may be configured such that drive piston 22 is inserted until cap 49 engages plunger closed end 33. Upon such complete insertion, elastic sealing member 62 engages and provides a seal against drive piston body 41 since piston body 41 has an outer diameter P, at the point of contact with elastic sealing member 62, which is larger than interior diameter d of elastic sealing member 62. Forward of the point of contact, piston body 41 is desirably narrower than interior diameter d of elastic sealing member 62 such that piston body 41 is inserted freely and air is not trapped between drive piston 22 and plunger 20.

The configuration and material of elastic sealing member 62 and the width of gap G are selected such that elastic sealing member 62 creates a friction force between plunger 20 and drive piston 22: The friction force is desirably designed to be greater than a pressure differential or the like which may act on the plunger 20, while still allowing easy separation of the components. In this regard, provision of elastic sealing member 62 reduces the potential pressure differential and thereby reduces the necessary friction force. For example, without elastic sealing member 62, a pressure differential may pass through gap G between plunger body 31 and drive piston body 41 and act on the complete surface S defined at closed end 33 of plunger 20. The force on plunger 20 relative to drive piston 22 which is fixed to lead screw 15 is equal to the pressure differential multiplied by the area upon which the pressure differential acts. The area of closed end 33 can be approximated as $A_{max\ plungerID} =$

$\Pi D^2/4$ wherein D is the inside diameter of plunger 20. In comparison, in the present invention, a pressure differential can only act on the elastic sealing member 62 which has a radial width only equal to the width of gap G. As such, the pressure differential only acts on an area of $A_{sealingmember} = \Pi(D^2-p^2)/4$ wherein P is the outside diameter of drive piston 22. Therefore, the ratio of the $A_{sealingmember}$ to that of the $A_{maxplunderID}$ is equal to

$$\frac{A_{sealingmember}}{A_{max\ plungerID}} = (D^2 - P^2)/D^2.$$ It is noted that a pressure differential will act on the rear end face of plunger 20, however, since this force will be equal in both scenarios and thereby cancel out, it has been removed from the equations.

In an illustrative example, if plunger inside diameter is 1 inch (2,54 cm) and drive piston outside diameter P is 90 percent thereof, or 0.9 inches (2,286 cm), the ratio of the $A_{sealingmember}$ to that of the $A_{maxplunderID}$

is equal to $\frac{A_{sealingmember}}{A_{max\ plungerID}} = (1^2 - 0.9^2)/1^2$ which

equals 0.19 or approximately one-fifth the area upon which the pressure differential can act. As a result, with a 10 percent clearance between drive piston 22 and plunger 20, elastic sealing member 62 reduces the resultant force created by the pressure differential by approximately 80 percent. Further reducing the clearance between drive piston 22 and plunger 20 will further reduce the resultant force created by the pressure differential. By lowering the resultant force, for example, by approximately 80 percent, the necessary friction force can also be reduced.

When syringe assembly 12 is to be removed, an axial force opposite arrow A is applied to syringe assembly 12. The axial force will be greater than the friction force created by elastic sealing member 62, however, as described above, the required axial force will be reduced in view of the reduced friction force. The configuration and material of elastic sealing member 62 and the width of gap G are selected to facilitate relatively easy axial insertion and removal of syringe assembly 12 while providing a sufficient friction force and minimizing the effect of a pressure differential force or the like.

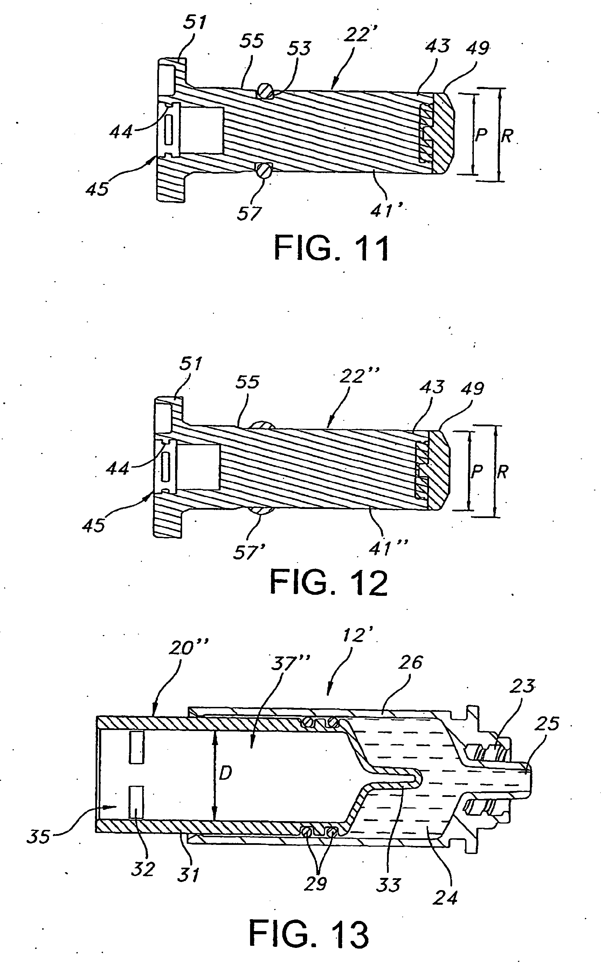

Referring to Figs. 11-13, alternative exemplary embodiments of the present invention are shown. The present embodiments are similar to the previous embodiments, except that elastic sealing member 57, 57' is provided on drive piston 22', 22" instead of on plunger 20" of syringe assembly 12". In each of these embodiments, plunger body 31 may be provided with hollow interior 37" which has a consistent diameter D without any inwardly extending portions.

With respect to drive piston 22' shown in Fig. 11, piston body 41' is provided with exterior annular groove 53 which is configured to receive sealing elastic element 57. Annular groove 53 is desirably provided proximate to shoulder 55. Elastic sealing member 57 and annular groove 53 are configured such that elastic sealing member 57 extends radially outward and has an outer diameter R which is larger than piston body diameter P and plunger inside diameter D. Upon insertion of drive piston 22', elastic sealing member 57 engages and seals against the inside surface of plunger hollow interior 37" and provides a reduced area for which a pressure differential or the like to act upon in a similar manner as described above with respect to the first two exemplary embodiments. In general aspects, the infusion pump assembly generally operates as in the previous embodiments.

In the exemplary embodiment illustrated in Fig. 12, elastic sealing member 57' is formed integrally with body 41" of drive piston 22", for example, via overmolding or the like. As in the previous embodiment, elastic sealing member 57' has an outside diameter R which is larger than piston body diameter P and plunger inside diameter D. In other respects, drive piston 22" operates in the same manner as described with respect to the above embodiments.

While the various embodiments have been illustrated and described with drive piston 22 extending Into plunger 20, the components may be reversed with a portion of the plunger extending into a portion of the drive piston.


**Claims**

1.  An infusion pump assembly (5) comprising a drive piston (22) and a syringe assembly, the syringe assembly (12) comprising:

    a substantially hollow syringe housing (26); and a plunger (20, 20') axially movable within the syringe housing to expel a fluid therefrom; the plunger (20, 20') having a plunger body (31) to engage the drive piston (22) with a gap (G) between the the plunger body (31) and drive piston (22), wherein the plunger body (31) is axially movable with respect to the drive piston (22) to engage and disengage the syringe assembly (12) from the infusion pump (5) via relative axial movement at any axial position of the plunger body (31) within the syringe housing (26), **characterized in that** a radially elastic sealing member (57, 57', 62, 62') is positioned within gap (G) to engage a portion of the drive piston aligned therewith to seal the plunger (20, 20') relative to the drive piston (22) whilst allowing relative axial movement therebetween so as to reduce the effective area of the plunger (20, 20') for a pressure differential to act upon.

2.  An infusion pump assembly according to claim 1 **characterized in that** the elastic sealing member (62, 62') is carried on the plunger body (31).

3.  The infusion pump assembly of any one of claims 1 or 2 **characterized in that** the plunger body (31) has a substantially hollow tubular configuration with an open end (35) configured to receive a portion of the drive piston (22) and the sealing elastic member (62, 62') extends from the plunger body (31) radially inwardly.

4.  The infusion pump assembly of any one of claims 1 to 3 **characterized in that** the elastic sealing member (62, 62') is provided on an insert (60) which is received and retained in an annular groove (39) on the plunger body (31).

5.  The infusion pump assembly of claim 1 or 2 **characterized in that** at least a portion of the plunger body (31) is configured to be received within a portion of the drive piston (22) and the elastic sealing member extends from the plunger body (31) radially outwardly.

6.  The infusion pump assembly of any one of claims 1 to 3 **characterized in that** the sealing elastic member (62, 62') is formed integrally with the plunger body (31).

7.  The infusion pump assembly of claim 1 **characterized in that** the elastic sealing member (62, 62') is positioned on the plunger body 31 such that the elastic sealing member is axially aligned with a forward portion of the drive piston (22) when the plunger (20, 20') is operationally positioned relative to drive piston (22).

8.  The infusion pump assembly of claim 1 **characterized in that** the elastic sealing member (57, 57') is carried on the drive piston (22).

9.  The infusion pump assembly of claim 1 or 8 **characterized in that** the elastic sealing member (57, 57') is formed integrally with the drive piston (22).

10. The infusion pump assembly of claim 8 **characterized in that** the drive piston (22) includes an annular groove (53) configured to receive and retain the elastic sealing member (57).

11. The infusion pump assembly of any one of claims 8 to 10 **characterized in that** the drive piston (22) includes a radial shoulder (55) configured to engage an open end (35) of the plunger (20, 20') when the plunger (20, 20') is operationally positioned relative to drive piston (22) and the elastic sealing member (57, 57') is axially positioned proximate the shoulder.

**12.** The infusion pump assembly of any one of claims 8 to 11 **characterized in that** the plunger body (31) has a substantially hollow tubular configuration with an open end (35) configured to receive a portion of the drive piston (22) and the elastic sealing member (57, 57') extends from the drive piston radially outwardly.

**13.** The infusion pump assembly of claim 1 **characterized in that** at least a portion of the plunger body (31) is configured to be received within a portion of the drive piston (22) and the elastic sealing member extends from the drive piston (22) radially inwardly.

**14.** The infusion pump assembly according to any one of claims 7 to 13 **characterized in that** the gap (G) is annular.

**15.** The infusion pump assembly of any one of claims claim 7 to 12 or 14 **characterized in that**, the plunger (31) has a hollow body including a closed end (33) having a given area (5) and the gap (G) has an annular area which is approximately one-fifth or less than the given area (5).

**Patentansprüche**

**1.** Infusionspumpenanordnung (5), umfassend einen Antriebskolben (22) und eine Spritzenanordnung, wobei die Spritzenanordnung (12) umfasst:

ein im wesentlichen hohles Spritzengehäuse (26); und
einen Kolben (20, 20'), der innerhalb des Spritzengehäuses axial bewegbar ist, um von dort eine Flüssigkeit auszustoßen;
wobei der Kolben (20, 20') einen Kolbenkörper (31) für einen Eingriff mit dem Antriebskolben (22) mit einer Lücke (G) zwischen dem Kolbenkörper (31) und Antriebskolben (22) aufweist, wobei der Kolbenkörper (31) bezüglich des Antriebskolbens (22) axial bewegbar ist, um die Spritzenanordnung (12) mit der Infusionspumpe (5) durch relative axiale Bewegung an jeder axialen Position des Kolbenkörpers (31) innerhalb des Spritzengehäuses (26) in Eingriff zu bringen und davon zu lösen, **dadurch** charakterisiert, dass ein radial elastisches Dichtungselement (57, 57', 62, 62') innerhalb der Lücke (G) angeordnet ist, um mit einem Abschnitt des Antriebskolbens, der damit ausgerichtet ist, in Eingriff zu treten, um den Kolben (20, 20') bezüglich des Antriebskolbens (22) abzudichten, während relative axiale Bewegung dazwischen ermöglicht wird, um die effektive Fläche des Kolbens (20, 20') zu reduzieren, damit ein Differenzdruck darauf ausgeübt werden kann.

**2.** Infusionspumpenanordnung nach Anspruch 1, **dadurch gekennzeichnet, dass** das abdichtende elastische Element (62, 62') auf dem Kolbenkörper (31) getragen wird.

**3.** Infusionspumpenanordnung nach einem der Ansprüche 1 oder 2, **dadurch gekennzeichnet, dass** der Kolbenkörper (31) eine im wesentlichen hohle röhrenförmige Konfiguration mit einem offenen Ende (35) aufweist, das konfiguriert ist, um einen Abschnitt des Antriebskolbens (22) aufzunehmen, und sich das abdichtende elastische Element (62, 62') von dem Kolbenkörper (31) radial nach innen erstreckt.

**4.** Infusionspumpenanordnung nach einem der Ansprüche 1 bis 3, **dadurch gekennzeichnet, dass** das abdichtende elastische Element (62, 62') an einem Einsatz (60) vorgesehen ist, der in einer Ringnut (39) auf dem Kolbenkörper (31) aufgenommen und gehalten wird.

**5.** Infusionspumpenanordnung nach Anspruch 1 oder 2, **dadurch gekennzeichnet, dass** wenigstens ein Abschnitt des Kolbenkörpers (31) konfiguriert ist, um innerhalb eines Abschnitts des Antriebskolbens (22) aufgenommen zu werden, und sich das abdichtende elastische Element von dem Kolbenkörper (31) radial nach außen erstreckt.

**6.** Infusionspumpenanordnung nach einem der Ansprüche 1 bis 3, **dadurch gekennzeichnet, dass** das abdichtende elastische Element (62, 62') mit dem Kolbenkörper (31) einteilig ausgebildet ist.

**7.** Infusionspumpenanordnung nach Anspruch 1, **dadurch gekennzeichnet, dass** das abdichtende elastische Element (62, 62') auf dem Kolbenkörper (31) so angeordnet ist, dass das abdichtende elastische Element mit einem vorderen Abschnitt des Antriebskolbens (22) axial ausgerichtet wird, wenn der Kolben (20, 20') im Betrieb relativ zu dem Antriebskolben (22) angeordnet wird.

**8.** Infusionspumpenanordnung nach Anspruch 1, **dadurch gekennzeichnet, dass** das abdichtende elastische Element (57, 57') auf dem Antriebskolben (22) getragen wird.

**9.** Infusionspumpenanordnung nach Anspruch 1 oder 8, **dadurch gekennzeichnet, dass** das abdichtende elastische Element (57, 57') mit dem Antriebskolben (22) einteilig ausgebildet ist.

**10.** Infusionspumpenanordnung nach Anspruch 8, **dadurch gekennzeichnet, dass** der Antriebskolben (22) eine Ringnut (53) aufweist, die konfiguriert ist, um das abdichtende elastische Element (57) aufzunehmen und zu halten.

**11.** Infusionspumpenanordnung nach einem der Ansprüche 8 bis 10, **dadurch gekennzeichnet, dass** der Antriebskolben (22) eine radiale Schulter (55) aufweist, die konfiguriert ist, um mit einem offenen Ende (35) des Kolbens (20, 20') in Eingriff zu treten, wenn der Kolben (20, 20') im Betrieb relativ zu dem Antriebskolben (22) angeordnet wird, und das abdichtende elastische Element (57, 57') neben der Schulter axial angeordnet wird.

**12.** Infusionspumpenanordnung nach einem der Ansprüche 8 bis 11, **dadurch gekennzeichnet, dass** der Kolbenkörper (31) eine im wesentlichen hohle röhrenförmige Konfiguration mit einem offenen Ende (35) aufweist, das konfiguriert ist, um einen Abschnitt des Antriebskolbens (22) aufzunehmen, und sich das abdichtende elastische Element (57, 57') von dem Antriebskolben radial nach außen erstreckt.

**13.** Infusionspumpenanordnung nach Anspruch 1, **dadurch gekennzeichnet, dass** wenigstens ein Abschnitt des Kolbenkörpers (31) konfiguriert ist, um innerhalb eines Abschnitts des Antriebskolbens (22) aufgenommen zu werden, und sich das abdichtende elastische Element von dem Antriebskolben (22) radial nach innen erstreckt.

**14.** Infusionspumpenanordnung nach einem der Ansprüche 7 bis 13, **dadurch gekennzeichnet, dass** die Lücke (G) ringförmig ist.

**15.** Infusionspumpenanordnung nach einem der Ansprüche 7 bis 12 oder 14, **dadurch gekennzeichnet, dass** der Kolben (31) einen hohlen Körper aufweist, der ein geschlossenes Ende (33) aufweist, das eine bestimmte Fläche (5) aufweist, und die Lücke (G) eine ringförmige Fläche aufweist, die etwa ein Fünftel oder weniger der bestimmten Fläche (5) aufweist.

**Revendications**

**1.** Ensemble de pompe à perfusion (5) comprenant un piston de commande (22) et un ensemble de seringue, l'ensemble de seringue (12) comprenant :

un logement de seringue sensiblement creux (26) et
un piston (20, 20') axialement mobile dans le logement de seringue pour en expulser un fluide ;
le piston (20, 20') ayant un corps de piston (31) pour mettre en prise le piston de commande (22) avec un intervalle (G) entre le corps de piston (31) et le piston de commande (22), dans lequel le corps de piston (31) est axialement mobile relativement au piston de commande (22) pour mettre en prise et dégager l'ensemble de seringue (12) de la pompe de perfusion (5), par le biais d'un mouvement axial relatif dans n'importe quelle position axiale du corps de piston (31) dans le logement de seringue (26), **caractérisé en ce que**
un élément d'étanchéité radialement élastique (57, 57', 62, 62') est positionné dans l'intervalle (G) pour mettre en prise une partie du piston de commande aligné avec lui, afin de sceller le piston (20, 20') relativement au piston de commande (22), tout en permettant un mouvement axial relatif entre eux, afin de réduire la superficie effective du piston (20, 20') pour qu'un différentiel de pression agisse dessus.

**2.** Ensemble de pompe à perfusion selon la revendication 1, **caractérisé en ce que** l'élément d'étanchéité élastique (62, 62') est supporté sur le corps de piston (31).

**3.** Ensemble de pompe à perfusion selon l'une quelconque des revendications 1 ou 2, **caractérisé en ce que** le corps de piston (31) a une configuration tubulaire sensiblement creuse avec une extrémité ouverte (35) configurée pour recevoir une partie du piston de commande (22) et l'élément élastique d'étanchéité (62, 62') s'étend depuis le corps de piston (31) radialement vers l'intérieur.

**4.** Ensemble de pompe à perfusion selon l'une quelconque des revendications 1 à 3, **caractérisé en ce que** l'élément élastique d'étanchéité (62, 62') est ménagé sur un insert (60), qui est reçu et retenu dans une cannelure annulaire (39) sur le corps de piston (31).

**5.** Ensemble de pompe à perfusion selon la revendication 1 ou 2, **caractérisé en ce qu'**au moins une partie du corps de piston (31) est configurée pour être reçue dans une partie du piston de commande (22) et l'élément d'étanchéité élastique s'étend depuis le corps de piston (31) radialement vers l'extérieur.

**6.** Ensemble de pompe à perfusion selon l'une quelconque des revendications 1 à 3, **caractérisé en ce que** l'élément d'étanchéité élastique (62, 62') est formé d'un seul tenant avec le corps de piston (31).

**7.** Ensemble de pompe à perfusion selon la revendication 1, **caractérisé en ce que** l'élément d'étanchéité élastique (62, 62') est positionné sur le corps de piston 31, de sorte que l'élément d'étanchéité élastique soit axialement aligné avec une partie vers l'avant du piston de commande (22), quand le piston (20, 20') est opérationnellement positionné relativement au piston de commande (22).

**8.** Ensemble de pompe à perfusion selon la revendication 1, **caractérisé en ce que** l'élément d'étanchéité élastique (57, 57') est supporté sur le piston de commande (22).

**9.** Ensemble de pompe à perfusion selon la revendication 1 ou 8, **caractérisé en ce que** l'élément d'étanchéité élastique (57, 57') est formé d'un seul tenant avec le piston de commande (22).

**10.** Ensemble de pompe à perfusion selon la revendication 8, **caractérisé en ce que** le piston de commande (22) comprend une cannelure annulaire (53) configurée pour recevoir et retenir l'élément d'étanchéité élastique (57).

**11.** Ensemble de pompe à perfusion selon l'une quelconque des revendications 8 à 10, **caractérisé en ce que** le piston de commande (22) comprend un épaulement radial (55) configuré pour mettre en prise une extrémité ouverte (35) du piston (20, 20'), quand le piston (20, 20') est opérationnellement positionné relativement au piston de commande (22) et l'élément d'étanchéité élastique (57, 57') est axialement positionné près de l'épaulement.

**12.** Ensemble de pompe à perfusion selon l'une quelconque des revendications 8 à 11, **caractérisé en ce que** le corps de piston (31) a une configuration tubulaire sensiblement creuse avec une extrémité ouverte (35), configurée pour recevoir une partie du piston de commande (22) et l'élément d'étanchéité élastique (57, 57') s'étend depuis le piston de commande radialement vers l'extérieur.

**13.** Ensemble de pompe à perfusion selon la revendication 1, **caractérisé en ce qu'**au moins une partie du corps de piston (31) est configurée pour être reçue dans une partie du piston de commande (22) et l'élément d'étanchéité élastique s'étend depuis le piston de commande (22) radialement vers l'intérieur.

**14.** Ensemble de pompe à perfusion selon l'une quelconque des revendications 7 à 13, **caractérisé en ce que** l'intervalle (G) est annulaire.

**15.** Ensemble de pompe à perfusion selon l'une quelconque des revendications 7 à 12 ou 14, **caractérisé en ce que** le piston (31) a un corps creux comprenant une extrémité fermée (33) ayant une superficie donnée (5) et l'intervalle (G) a une surface annulaire qui est approximativement d'un cinquième ou moins de la superficie donnée (5).

## FIG. 1

## FIG. 2

## FIG. 3

FIG. 4

FIG. 5

FIG. 6

FIG. 7

FIG. 8

FIG. 9

FIG. 10

**FIG. 11**

**FIG. 12**

**FIG. 13**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2895773 A
- DE 20011366 U1
- WO 9955401 A
- WO 0204049 A